# EUROPÄISCHE PATENTSCHRIFT

(11) **EP 1 765 343 B1**
(45) Veröffentlichungstag und Bekanntmachung des Hinweises auf die Patenterteilung: **28.10.2009**
(21) Anmeldenummer: 05753010.7
(22) Anmeldetag: 23.06.2005
(51) Int. Cl.: A61K 31/4427, A61P 1/08

(54) **VERWENDUNG VON SUBSTITUIERTEN 2-THIO-3,5-DICYANO-4-PHENYL-6-AMINOPYRIDINEN BEI DER BEHANDLUNG VON ÜBELKEIT UND ERBRECHEN**
USE OF SUBSTITUTED 2-THIO-3,5-DICYANO-4-PHENYL-6-AMINOPYRIDINES IN THE TREATMENT OF NAUSEA AND VOMITING
UTILISATION DE 2-THIO-3,5-DICYANO-4-PHENYL-6-AMINOPYRIDINES SUBSTITUEES LORS DU TRAITEMENT DE NAUSEES ET DE VOMISSEMENTS

(30) Priorität: 06.07.2004 DE 102004032651
(43) Veröffentlichungstag der Anmeldung: 28.03.2007
(73) Patentinhaber: Bayer Schering Pharma Aktiengesellschaft, 13353 Berlin (DE)
(72) Erfinder: KRAHN, Thomas, 58135 Hagen (DE); THIELEMANN, Wolfgang, 42107 Wuppertal (DE); ROSENTRETER, Ulrich, 42349 Wuppertal (DE); DIEDRICHS, Nicole, 42553 Velbert (DE); KRÄMER, Thomas, 42111 Wuppertal (DE)
(86) Internationale Anmeldenummer: PCT/EP2005/006779
(87) Internationale Veröffentlichungsnummer: WO 2006/002823

(56) Entgegenhaltungen:
- WO-A-03/008384
- WO-A-03/053441

## Beschreibung

Die vorliegende Erfindung betrifft die Verwendung von substituierten 2-Thio-3,5-dicyano-4-phenyl-6-aminopyridinen der Formel (I-A) und (I-B) zur Herstellung eines Arzneimittels zur Prophylaxe und/oder Behandlung von Übelkeit und Erbrechen.

Es ist bekannt, dass sowohl Adenosinphosphate als auch Adenosin selbst im allgemeinen zu einer schnelleren Erholung von Patienten führen, wenn sie nach einer Narkose verabreicht werden. Dabei wird beobachtet, dass so behandelte Patienten auch weniger unter Übelkeit und Erbrechen leiden.

Übelkeit und Erbrechen können unter anderem hervorgerufen werden durch eine medikamentöse Therapie, beispielsweise Chemotherapie zur Behandlung von Tumoren mit alkylierenden Substanzen wie beispielsweise Altretamin, Busulfan, Carmustin, Chlorambucil, Cyclophosphamid, Cytoxan, Dacarbazin, Estramustinephosphat, Fotemustin, Ifosfamid, Lomustin, Melphalan, Miltefosin, Nimustin, Procarbazin, Streptozocin, Temozolomid, Thiotepa und Trofosfamid; mit zytotoxischen Antibiotika wie beispielsweise Azacitidin, Bleomycin, Dactinomycin, Daunorubicin, Doxorubicin, Epirubicin, Idarubicin, Mitomycin, Mitoxantron, Neocarzinostatin, Pirarubicin und Valrubicin; mit Antimetaboliten wie beispielsweise Capecitabin, Carmofur, Cladribin, Clofarabin, Cytarabin, Decarbazin, Doxifluridin, Floxuridin, Fludarabinephosphat, Fluoruracil, Folinsäure, Gemcitabin, Leucovorin, Masoprocol, Mercaptopurin, Methotrexat, Pemetrexed, Pentostatin, Raltitrexed und Tegafur; mit Alkaloiden wie beispielsweise Docetaxel, Etoposid, Irinotecan, Paclitaxel, Teniposid, Topetecan, Topotecan, Vinblastin, Vincristin, Vindesin und Vinorelbin; oder mit anderen Chemotherapeutika wie beispielsweise Carboplatin, Cisplatin, Hydroxyharnstoff, Lobaplatin, Nedaplatin und Oxaliplatin, sowie Kombinationen dieser; bei der Chemotherapie von bakteriellen Infektionen mit Sulfonamid-Antibiotika wie beispielsweise Sulfamethoxazol und Sulfisoxazol; mit Makrolid-Antibiotika wie beispielsweise Erythromycin, Azithromycin, Clarithromycin und Dirithromycin; mit Fluorchinolon-Antibiotika wie beispielsweise Ciprofloxacin, Levofloxacin und Gatifloxacin; mit Oxazolidinon-Antibiotika wie beispielsweise Linezolid; bei der Chemotherapie von viralen Infektionen mit antiviralen Wirkstoffen wie beispielsweise Abacavir, Didanosin, Emtricitabin, Indinavir, Tenofovir, Zalacitabin, Zidovudin, Delaviridin, Amprenavir, Fosamprenavir, Lopinavir, Nelfinavir, Ritonavir, Saquinavir, Stavudin und Acyclovir; oder bei der Therapie von Depressionen mit Monaminoxidase-Inhibitoren wie beispielsweise Selegilin, Isocarboxazid und Tranylcyprominsulfat; bei der Behandlung von Atemwegserkrankungen wie COPD mit Inhibitoren des Enzyms PDE 4 wie beispielsweise Cilomilast oder Roflumilast; durch das Bestrahlungssyndrom, Strahlentherapie, Bestrahlung des Brustkorbs oder Unterleibs, wie beispielsweise bei der Behandlung von Krebs; durch Gifte und Giftstoffe, wie sie beispielsweise durch Stoffwechselkrankheiten oder Infektionen entstehen können (z.B. Magenschleimhautentzündung); bei Schwangerschaft; durch vestibuläre Störungen wie beispielsweise Bewegungsübelkeit oder Schwindel, durch Übelkeit in Folge einer Operation, sowie Magen-Darm-Verstopfung; reduzierte Magen-Darm-Aktivität, durch viszeralen Schmerz, wie beispielsweise bei Herzinfarkt oder Bauchfellentzündung; durch Migräne; durch erhöhten oder erniedrigten intracraniellen Druck wie beispielsweise bei Höhenkrankheit und durch opioide Analgetika wie Morphin.

In WO 02/069982 A1 wird die antiemetische Wirkung von A1-Agonisten, vorzugsweise von partiellen A1-Agonisten, am Beispiel Adenosin-analoger Strukturen beschrieben.

Überraschenderweise wurde nun gefunden, dass sowohl spezifische als auch unspezifische nicht-Adenosin-analoge Adenosinagonisten zur Herstellung von Medikamenten zur Prophylaxe und/oder Behandlung von Übelkeit und Erbrechen bei Säugetieren, insbesondere bei Menschen, geeignet sind.

Dieses gilt vorzugsweise für die Verbindungen der Formel (I-A) und (I-B), deren Herstellung und Verwendung als Arzeimittel, insbesondere für die Behandlung von kardiovaskulären Erkrankungen in WO 03/053441 und WO 03/008384 ausführlich beschrieben worden ist. Die dort im allgemeinen und vor allem die dort spezifisch genannten Verbindungen sind ausdrücklicher Beschreibungsbestandteil der vorliegenden Erfindung.

### Die Verbindung der Formeln (I-A) und (I-B) zeichnen sich durch ihre Wirkung als nicht-Adenosinanaloge Adenosin-Agonisten aus.

Die Verbindungen der Formeln (I-A) und (I-B) wirken sowohl als A1-spezifische (Adenosin A1-agonistische Wirkung um den Faktor 10 größer im Vergleich zu dem agonistischen Effekt auf die übrigen Adenosinrezeptoren, A2a, A2b und A3) als auch als A1-unspezifische (mindestens ein weiterer agonistischer Effekt auf einen der übrigen Adenosinrezeptoren A2a, A2b oder A3, welcher sich nicht um den Faktor 10 von dem A1-agonistischen Effekt unterscheidet), Adenosinagonisten.

Gegenstand der vorliegenden Erfindung ist die Verwendung von Verbindungen der Formel (I-A) 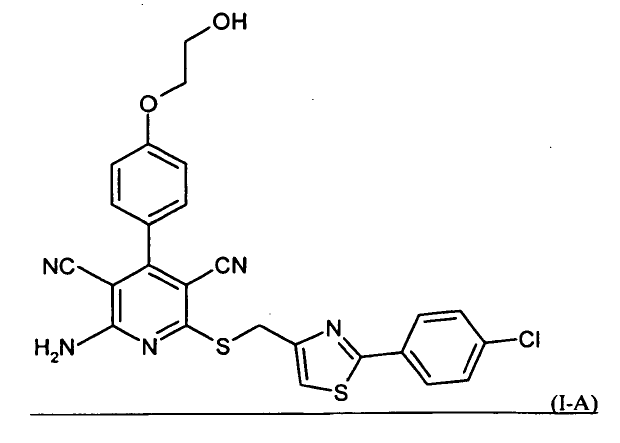 oder (I-B) 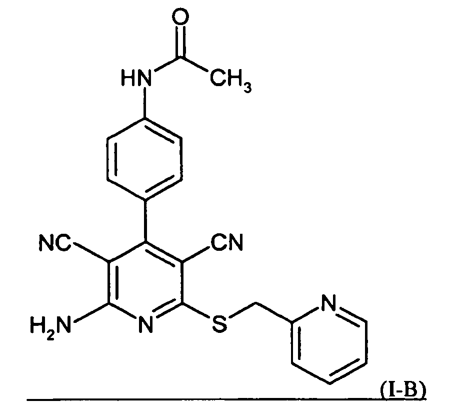 und ihrer Salze, Hydrate, Hydrate der Salze und Solvate zur Herstellung eines Arzneimittels zur Prophylaxe und/oder Behandlung von Übelkeit und Erbrechen.

Besonders bevorzugt ist die erfindungsgemäße Verwendung der Verbindung der Formel (I-A) (entsprechend Beispiel 6 aus WO 03/053441) 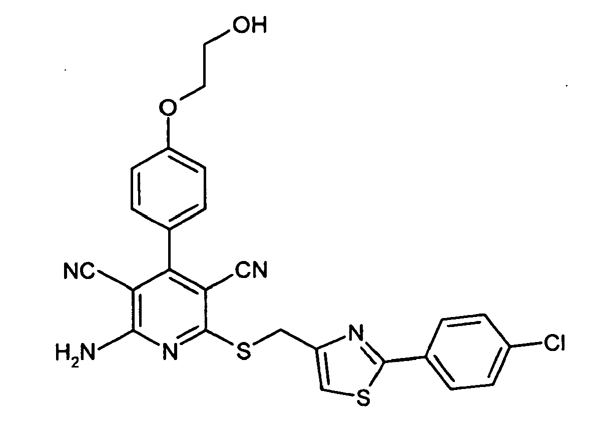 und ihrer Salze, Hydrate, Hydrate der Salze und Solvate.

Ebenfalls besonders bevorzugt ist die erfindungsgemäße Verwendung der Verbindung der Formel (I-B) (entsprechend Beispiel 1 aus WO 03/008384) 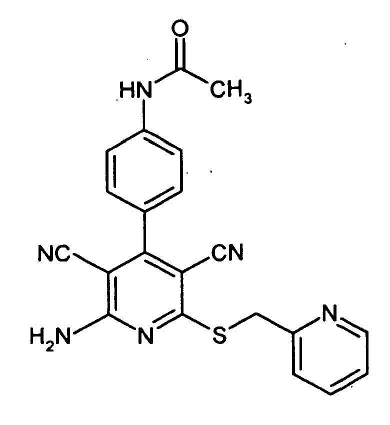 und ihrer Salze, Hydrate, Hydrate der Salze und Solvate.

Die Verbindungen der Formeln (I-A) und (I-B) können in Abhängigkeit vom Substitutionsmuster in stereoisomeren Formen, die sich entweder wie Bild und Spiegelbild (Enantiomere) oder die sich nicht wie Bild und Spiegelbild (Diastereomere) verhalten, existieren. Die Erfindung betrifft sowohl die Verwendung der Enantiomeren oder Diastereomeren als auch deren jeweilige Mischungen. Die Racemformen lassen sich ebenso wie die Diastereomeren in bekannter Weise in die stereoisomer einheitlichen Bestandteile trennen. Gleichermaßen betrifft die vorliegende Erfindung auch die Verwendung der übrigen Tautomeren der Verbindungen der Formel (I) und deren Salze.

Salze der Verbindungen der Formeln (I-A) und (I-B) können physiologisch unbedenkliche Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Trifluoressigsäure, Essigsäure, Propionsäure, Milchsäure, Weinsäure, Zitronensäure, Fumarsäure, Maleinsäure oder Benzoesäure.

Als Salze können auch Salze mit üblichen Basen genannt werden, wie beispielsweise Alkalimetallsalze (z.B. Natrium- oder Kaliumsalze), Erdalkalisalze (z.B. Calcium- oder Magnesiumsalze) oder Ammoniumsalze, abgeleitet von Ammoniak oder organischen Aminen wie beispielsweise Diethylamin, Triethylamin, Ethyldiisopropylamin, Prokain, Dibenzylamin, N-Methylmorpholin, Dihydroabietylamin, 1-Ephenamin oder Methylpiperidin.

Als Hydrate bzw. Solvate werden erfindungsgemäß solche Formen der Verbindungen der Formeln (I-A) und (I-B) bezeichnet, welche in festem oder flüssigem Zustand durch Hydratation mit Wasser oder Koordination mit Lösungsmittelmolekülen eine Molekül-Verbindung bzw. einen Komplex bilden. Beispiele für Hydrate sind Sesquihydrate, Monohydrate, Dihydrate oder Trihydrate. Gleichermaßen kommen auch die Hydrate bzw. Solvate von Salzen der erfindungsgemäßen Verbindungen in Betracht.

Außerdem umfasst die Erfindung auch die Verwendung von Prodrugs der Verbindungen der Formeln (I-A) und (I-B). Als Prodrugs werden erfindungsgemäß solche Formen der Verbindungen der Formeln (I-A) und (I-B) bezeichnet, welche selbst biologisch aktiv oder inaktiv sein können, jedoch unter physiologischen Bedingungen in die entsprechende biologisch aktive Form überführt werden können (beispielsweise metabolisch oder solvolytisch).

Die vorliegende Erfindung betrifft weiterhin eine Verbindung der Formel (I-A) oder (I-B) zur Verwendung in einem Verfahren zur Prophylaxe und/oder Behandlung von Übelkeit und Erbrechen.

In der medikamentösen Therapie ist die bevorzugte Anwendung die Prophylaxe (so die Substanzgabe, bevor der Patient einem bekannten Stimulus für Übelkeit und Erbrechen ausgesetzt wird, z.B. Chemotherapie, Bestrahlung, Vollnarkose).

Weiterer Gegenstand der vorliegenden Erfindung ist eine pharmazeutische Zusammensetzung, enthaltend eine Verbindung der Formeln (I-A) oder (I-B).

Für die Applikation der Verbindungen der Formeln (I-A) und (I-B) kommen alle üblichen Applikationsformen in Betracht, d.h. also oral, parenteral, inhalativ, nasal, sublingual, rektal, lokal wie z.B. bei Implantaten oder Stents, oder äußerlich wie z.B. transdermal. Bei der parenteralen Applikation sind insbesondere intravenöse, intramuskuläre, subkutane Applikation zu nennen, z.B. als subkutanes Depot.

Aufgrund der pharmakokinetischen Eigenschaften der Verbindungen der Formeln (I-A) und (I-B) ist ihre erfindungsgemäße Verwendung bei der oralen Therapie bevorzugt.

Die Wirkstoffe können allein oder in Form von Zubereitungen verabreicht werden. Für die orale Applikation eignen sich als Zubereitungen u.a. Tabletten, Kapseln, Pellets, Dragees, Pillen, Granulate, feste und flüssige Aerosole, Sirupe, Emulsionen, Suspensionen und Lösungen. Hierbei muss der Wirkstoff in einer solchen Menge vorliegen, dass eine therapeutische Wirkung erzielt wird.

Die Dosis und/oder Formulierung ist u. a. auch abhängig von der zugrunde liegenden Ursache, dem Alter und der Verfassung des Patienten und liegt schließlich im Ermessen des Arztes, Apothekers oder Tierarztes. Im allgemeinen, wird die Dosis um eine erwachsenen Menschen zu behandeln im Bereich von 0,01 bis 5000 mg pro Tag liegen, bevorzugt 0,5 bis 1000 mg pro Tag. Die Tagesdosis kann dabei als einzelne Dosis oder in Form mehrer Teildosen in entsprechenden Intervallen verabreicht werden, beispielsweise als zwei, drei, vier oder mehr Teildosen pro Tag.

Die Formulierungen können dabei entsprechend des Eingriffes aktive Substanz zwischen 0,1 und 99 % Wirkstoff enthalten, in geeigneter Weise 25-95 % bei Tabletten und Kapseln und 1-50 % bei flüssigen Formulierungen d.h. der Wirkstoff sollte in Mengen vorliegen, die ausreichend sind, den angegebenen Dosierungsspielraum zu erreichen.

Zu diesem Zweck können die Wirkstoffe in an sich bekannter Weise in die üblichen Zubereitungen überführt werden. Dies geschieht unter Verwendung inerter, nichttoxischer, pharmazeutisch geeigneter Trägerstoffe, Hilfsstoffe, Lösungsmittel, Vehikel, Emulgatoren und/oder Dispergiermittel.

Als Hilfsstoffe seien beispielsweise aufgeführt: Wasser, nichttoxische organische Lösungsmittel wie z.B. Paraffine, pflanzliche Öle (z.B. Sesamöl), Alkohole (z.B. Ethanol, Glycerin); Glykole (z.B. Polyethylenglykol), feste Trägerstoffe wie natürliche oder synthetische Gesteinsmehle (z.B. Talkum oder Silikate), Zucker (z.B. Milchzucker), Emulgiermittel, Dispergiermittel (z.B. Polyvinylpyrrolidon) und Gleitmittel (z.B. Magnesiumsulfat).

Im Falle der oralen Applikation können Tabletten selbstverständlich auch Zusätze wie Natriumcitrat zusammen mit Zuschlagstoffen wie Stärke, Gelatine und dergleichen enthalten. Wässrige Zubereitungen für die orale Applikation können weiterhin mit Geschmacksaufbesserern oder Farbstoffen versetzt werden.

Weiterer Gegenstand der vorliegenden Erfindung ist die Verwendung einer Kombination einer oder mehrerer Verbindungen der Formeln (I-A) oder (I-B) mit einem oder mehreren anderen Wirkstoffen. Geeignete Kombinationswirkstoffe sind beispielsweise andere Wirkstoffe, die zur Prophylaxe und/oder Behandlung von Übelkeit und Erbrechen geeignet sind. Beispielhaft und vorzugsweise seine 5HT3-Antagonisten genannt, wie z.B. Ondansetron, Granisetron, Palonosetron, Tropisetron, Ramosetron. Weiterhin sind die hier beschriebenen Adenosinagonisten für die Kombination mit Neurokinin-Antagonisten, Dopamin-Antagonisten, Cannabinoiden und anderen Therapien zur Prophylaxe und/oder Behandlung von Übelkeit und Erbrechen geeignet.

### Experimenteller Teil:

### Emetin-induzierter Brechreiztest am Frettchen:

Die Bestimmung der antiemetische Wirkung folgt der von Gardner et al. in Brit. J. Pharmacol., 116, 3158-3163, 1995 beschriebenen Methode.

Sechzig Minuten vor Gabe der Testsubstanz, wurden die Frettchen in individuellen Inox-Käfigen (40 x 50 x 34 cm) mit Gitterboden untergebracht. Dann wurden die Tiere mit Emetin (2 mg/kg p.o.) behandelt und unverzüglich über einen Zeitraum von 2 Stunden mit Blick auf folgende Punkte beobachtet:
- Zahl der Frettchen, die Anzeichen für Würgen und Erbrechen geben;
- Latenz bis zum ersten Würgen (Stunden, Minuten, Sekunden);
- Latenz bis zum ersten Erbrechen (Stunden, Minuten, Sekunden);
- Würgen (wie oft);
- Erbrechen (wie oft);
- Anzahl der Übelkeiten;
- durchschnittliche Dauer der Erbrechenszeiträume (Minuten, Sekunden);
- schwerwiegende Nebenwirkungen auf das Verhalten.

Würgen ist erfindungsgemäß definiert als eine rhythmische Atembewegung gegen geschlossene Stimmbänder, während Erbrechen erfindungsgemäß definiert ist als erzwungene Ausscheidung höheren Magen- und Darminhalts.

Es wurden 32 Frettchen untersucht. Die getestete Substanz wurde in zwei Dosierungen verabreicht, oral sechzig Minuten vor der Emetin-Gabe, und gegen eine Kontrollgruppe verglichen. Ondansetron (16 mg/kg p.o.) wurde unter den gleichen experimentellen Bedingungen gegeben und diente als Referenzsubstanz.

Die 32 Frettchen wurden wiederholt eingesetzt, um 4 Testsubstanzen zu prüfen. Das Applikationsschema für den dreiwöchigen Versuch sah folgendermaßen aus:
Woche 1:
   8 Frettchen für Lösungsmittelkontrolle
   8 Frettchen für Dosis 1 von Substanz 1
   8 Frettchen für Dosis 2 von Substanz 1
   8 Frettchen für Referenzsubstanz
Woche 2:
   8 Frettchen für Dosis 1 von Substanz 2
   8 Frettchen für Dosis 2 von Substanz 2
   8 Frettchen für Dosis 1 von Substanz 3
   8 Frettchen für Dosis 2 von Substanz 3
Woche 3:
   8 Frettchen für Lösungsmittelkontrolle
   8 Frettchen für Dosis 1 von Substanz 4
   8 Frettchen für Dosis 2 von Substanz 4
   8 Frettchen für Referenzsubstanz

Die Mengenangaben wurden analysiert und beim Vergleich der behandelten Gruppen mit der Kontrollgruppe der Student-Test angewendet.

Die quantitativen Angaben wurden analysiert, wobei zum Vergleich der behandelten Gruppen mit der Kontrollgruppe Fisher's Exact-Wahrscheinlichkeitstest verwendet wurde.

### Ergebnis:

Die Referenzsubstanz Ondansetron (16 mg/kg p.o.) hat signifikant das quantitative Auftreten von Würgen und Erbrechen (induziert durch Emetin) reduziert. Emesis wurde nur in einem von 16 Frettchen induziert.

Substanz 1 und Substanz 2 hatten in einer Dosis von 0,3 und 3,0 mg/kg p.o. keinen signifikanten anti-emetischen Effekt auf Frettchen, denen Emetin gegeben wurde. Substanz 3 und Substanz 4 dagegen haben signifikant das Emetin-indizierte Erbrechen verhindert. Die Gabe von 0,3 und 3,0 mg/kg p.o. von Substanz 4, gegeben 60 Minuten vor der Emetin-Gabe, hat signifikant das quantitative Auftreten von Würgen und Erbrechen (induziert durch Emetin) reduziert. Emesis wurde in keinem der mit 3,0 mg/kg p.o. behandelten Frettchen induziert.

### Strukturen der Substanzen 1 bis 4:

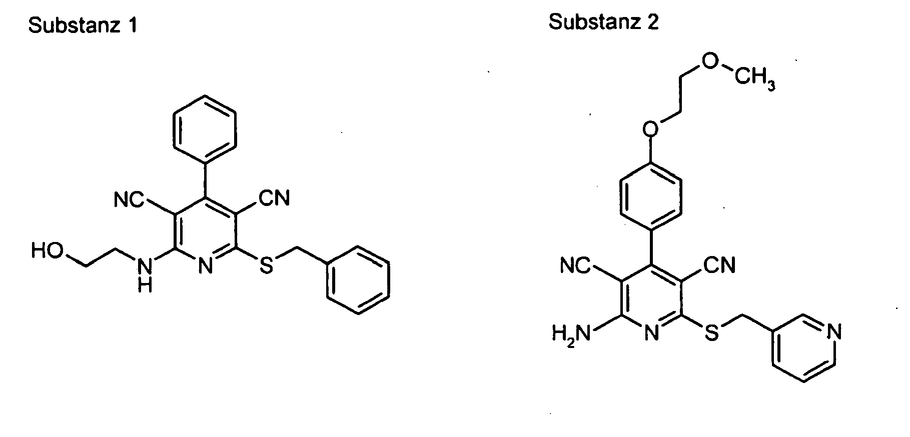

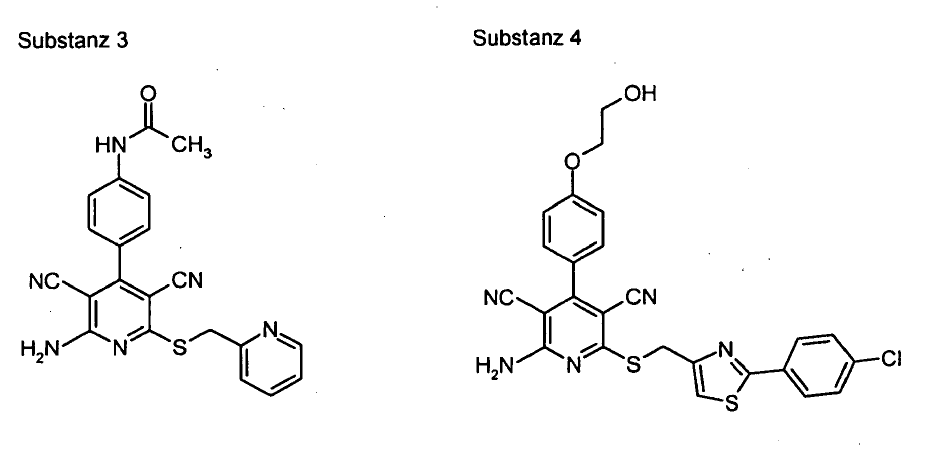

## Patentansprüche

1. Verwendung einer Verbindung der Formel (I-A) 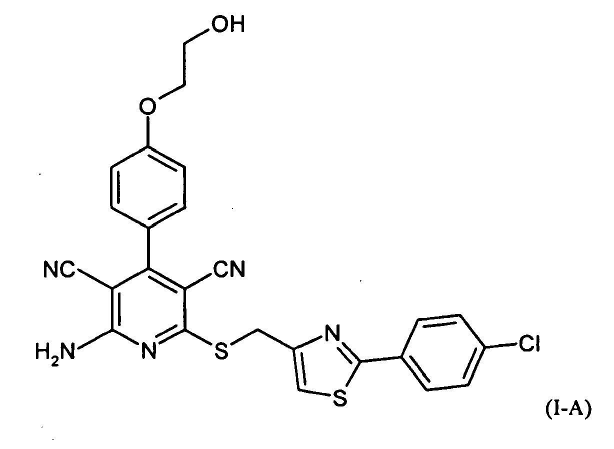 oder (I-B) 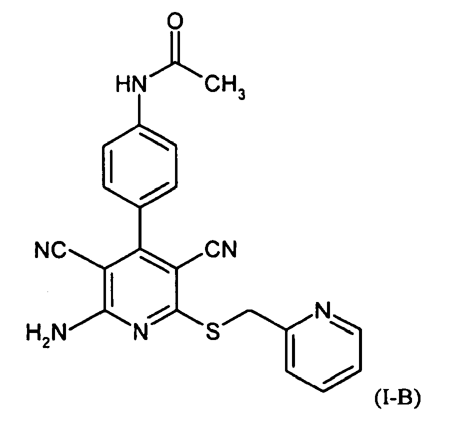 und ihrer Salze, Hydrate, Hydrate der Salze und Solvate zur Herstellung eines Arzneimittels zur Prophylaxe und/oder Behandlung von Übelkeit und Erbrechen.

2. Verwendung nach Anspruch 1, **dadurch gekennzeichnet, dass** das Arzneimittel oral angewendet wird.

3. Verwendung nach Anspruch 1, **dadurch gekennzeichnet, dass** das Arzneimittel zur Prophylaxe angewendet wird.

4. Verbindung der Formel (I-A) oder (I-B) zur Verwendung in einem Verfahren zur Behandlung von Übelkeit und Erbrechen.

## Claims

1. Use of a compound of the formula (I-A) 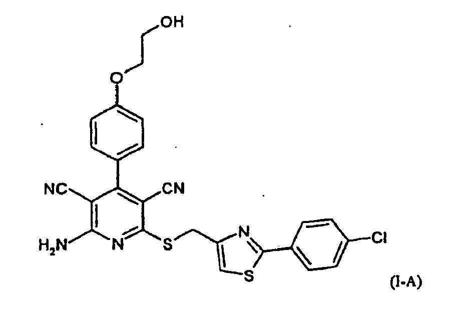 or (I-B) 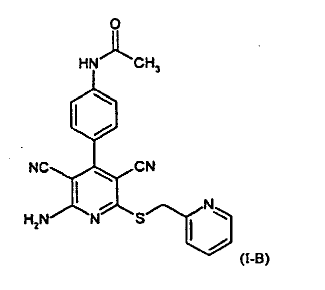 and its salts, hydrates, hydrates of the salts and solvates for production of a medicament for the prophylaxis and/or treatment of nausea and vomiting.

2. Use according to Claim 1, **characterized in that** the medicament is administered orally.

3. Use according to Claim 1, **characterized in that** the medicament is used for prophylaxis.

4. Compound of the formula (I-A) or (I-B) for use in a method for the treatment of nausea and vomiting.

## Revendications

1. Utilisation d'un composé de formule (I-A) 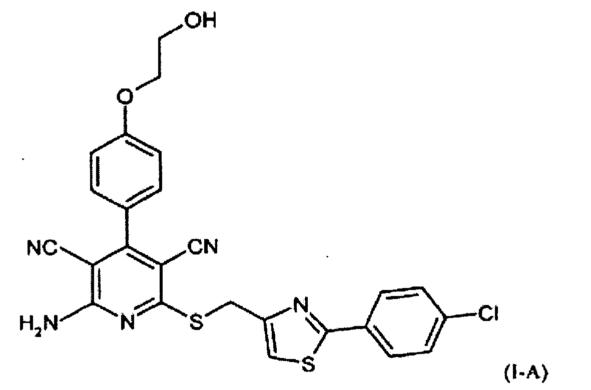 ou (I-B) 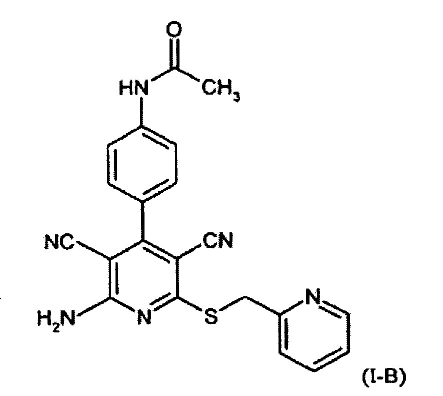 et de ses sels, hydrates, hydrates des sels et produits de solvatation pour la préparation d'un médicament destiné à la prophylaxie et/ou au traitement de la nausée et des vomissements.

2. Utilisation suivant la revendication 1,
**caractérisée en ce que** le médicament est administré par voie orale.

3. Utilisation suivant la revendication 1,
**caractérisée en ce que** le médicament est administré à des fins prophylactiques.

4. Composé de formule (I-A) ou (I-B) destiné à être utilisé dans une méthode de traitement de la nausée et des vomissements.
